# EUROPÄISCHE PATENTSCHRIFT

(11) **EP 1 103 274 B1**
(45) Veröffentlichungstag und Bekanntmachung des Hinweises auf die Patenterteilung: **09.11.2005**
(21) Anmeldenummer: 00125083.6
(22) Anmeldetag: 17.11.2000
(51) Int. Cl.: A61L 9/014, B01D 53/02

(54) **Absorption von Formaldehyd in geschlossenen, gasundurchlässigen Gebinden**
Absorption of formaldehyde in closed gas impervious packages
Absorption de Formaldehyde dans des emballages fermés et non perméables aux gaz

(30) Priorität: 29.11.1999 DE 19957439
(43) Veröffentlichungstag der Anmeldung: 30.05.2001
(73) Patentinhaber: Ticona GmbH, 65451 Kelsterbach (DE)
(72) Erfinder: Disch, Stefan, Dr., 60599 Frankfurt (DE); Vaahs, Tilo, Dr., 65510 Idstein (DE)

(56) Entgegenhaltungen:
- WO-A-93/02130
- US-A- 5 413 827
- US-A- 5 942 323

## Beschreibung

Die Erfindung betrifft ein Verfahren zur Absorption von Formaldehyd in geschlossenen, gasundurchlässigen Gebinden sowie die Verwendung einer speziellen Metalloxid und Permanganat enthaltenden Mischung zur Entfernung von Formaldehyd aus dem eingeschlossenen Gasraum von geschlossenen, gasundurchlässigen Gebinden. Das erfindungsgemäße Verfahren ist auf Verpackungen von formaldehydfreisetzenden Kunststoffen, wie Polyacetale, Phenol-Formaldehydharze (Bakelit) oder Melamin-Formaldehydharze anwendbar, insbesondere aber auf acetalhaltige Kunststoffe.

Granulat und Formteile aus Polyacetal enthalten nach ihrer Herstellung oft noch Restbestandteile von flüchtigen Substanzen oder Spaltungsprodukten, insbesondere Formaldehyd, die mit der Zeit aus dem Granulat oder dem Formteil heraus diffundieren. Sofern möglich, werden deshalb Granulate oder Formteile zum Auslüften an freier Luft gelagert. Für manche Anwendungen, insbesondere im medizinischen Sektor, ist es jedoch zwingend notwendig, das hergestellte Formteil oder eine Baugruppe mit einem oder mehreren Formteilen aus Polyacetal umgehend in eine geschlossene Verpackung zu überführen, wobei häufig gasundurchlässige Verpackungsmaterialien verwendet werden.

Ganz gleich, ob es sich bei diesen Verpackungen um mit Luft, einem Gas oder einer Gasmischung gefüllte Verpackungen oder um gasleere Vakuumverpackungen handelt, diffundieren die oben genannten flüchtigen Substanzen und Spaltungsprodukte mit der Zeit aus dem Granulat oder dem Formteil heraus und reichern sich im Innenraum der Verpackung an. Wird die Verpackung zu einem späteren Zeitpunkt geöffnet, entweichen die angereicherten flüchtigen Substanzen und Spaltungsprodukte schlagartig und verbreiten dabei in der Regel einen mehr oder weniger starken unangenehmen Geruch.

Ähnliche Probleme treten bei Verpackungen anderer polyacetalhaltiger oder formaldehydfreisetzender Objekte oder Materialien in geschlossenen, gasundurchlässigen Verpackungen (= Gebinden) auf.

Es bestand nun die Aufgabe, eine Möglichkeit zu finden, polyacetalhaltige oder formaldehydfreisetzende Objekte oder Materialien so in geschlossenen, gasundurchlässigen Gebinden zu verpacken, daß sich flüchtige Substanzen und Spaltungsprodukte, insbesondere Formaldehyd, während der Lagerungszeit innerhalb des Gasraumes der Verpackung nicht anreichern.

Aus den US-Patenten 5284892 und 5413827 ist die Verwendung von Polyalkylimiden, insbesondere Polyethylenimiden (PAI) als Formaldehydfänger für Lebensmittelverpackungen bekannt. Dabei wird das PAI, wahlweise zusammen mit einem Sauerstoffänger, vorzugsweise in das aus einem olefinischen Polymer bestehende Verpackungsmaterial eingearbeitet.

Kommerziell erhältlich ist ein kugelförmiges Gas-Filtrations-Material (PURATEX; ATEX Filter, Sprockhövel) aus aktiviertem Aluminiumoxid und Kaliumpermanganat, das zur Beseitigung von gasförmigen Schadstoffen im niedrigen ppm-Bereich dient. Dazu wird das Filtermaterial in Filtermodule gefüllt, durch die das Rohgas geleitet und durch Oxidation von Schadstoffen befreit wird.

Bekannt ist auch die Entfernung von gasförmigen Silikonverbindungen durch aktiviertes Aluminiumoxid (Al₂O₃) (JP 6269631 A) sowie die Entfernung von Stickoxiden mittels Lösungen enthaltend Alkalimetallmanganate oder -permanganate (US 4001372).

Die Aufgabe wurde dahingehend gelöst, daß kleine Mengen einer Absorbermischung enthaltend aktiviertes Aluminiumoxid und Kaliumpermanganat in den Innenraum der Verpackung gegeben werden.

Die Erfindung betrifft daher ein Verfahren zur Desodorierung des Innenraums von geschlossenen, gasundurchlässigen Gebinden, das dadurch gekennzeichnet ist, daß in das Gebinde eine geringe Menge einer Absorbermischung enthaltend aktiviertes Aluminiumoxid und Kaliumpermanganat gegeben wird.

Unter Desodorierung wird dabei die Entfernung (= Beseitigung, Absorption) von aus Kunststofformteilen oder -materialien austretenden flüchtigen Bestandteilen, insbesondere Spaltungsprodukte wie Formaldehyd verstanden.

Die Erfindung betrifft besonders die Desodorierung des Innenraums von Gebinden, die polyacetalhaltige oder sonstige formaldehydfreisetzende Materialien oder Objekte beinhalten.

Überraschend wurde festgestellt, daß bereits eine geringe Menge des kommerziell erhältlichen Gas-Filtrations-Materials PURATEX genügt, um in geschlossenen, nicht durchströmten gasundurchlässigen Gebinden nicht nur eine Anreicherung von flüchtigen oxidierbaren Bestandteilen, insbesondere Spaltungsprodukte wie Formaldehyd, zu verhindern, sondern gar die Konzentration des Formaldehyds im Gebinde deutlich zu verringern. Bisher wird PURATEX als Filtrationsmedium in durchströmten Filtrationsanordnungen eingesetzt. PURATEX wird dabei zur Beseitigung oxidierbarer gasförmiger Verbindungen verwendet, z.B. zur Beseitigung von Ethanol, Ameisensäure, Ammoniak, Formaldehyd, Schwefeldioxid oder Stickstoffmonoxid.

Allgemein können auch andere Materialien aus einer Mischung enthaltend aktiviertes Aluminiumoxid und Kaliumpermanganat eingesetzt werden. Unter aktiviertem Aluminiumoxid werden poröse Aluminiumoxid-Partikel verstanden, die einen Wassergehalt von 0 bis 0,5 Gew.-% aufweisen. Diese Mischungen können zudem auch Aktivkohle, Wasserabsorber und/oder weitere Formaldehydfänger wie Polyalkylenimide etc. enthalten. Vorzugsweise enthalten die Mischungen aktiviertes Aluminiumoxid in einer Menge von 80 bis 99,999 Gew.-%, Kaliumpermanganat in einer Menge von 0,001 bis 20 Gew.-% und Aktivkohle in einer Menge von 0 bis 10 Gew.-% bezogen auf die Mischung von Aluminiumoxid und Kaliumpermanganat.

Die Mischungen können, wahlweise mit Bindemittel versetzt, zu Granulat verarbeitet oder verpreßt werden. Ein Granulat, beispielsweise bestehend aus aktiviertem Aluminiumoxid und Aktivkohle in einem Mischungsverhältnis im Sinne der Erfindung, kann auch in eine wäßrige Kaliumpermanganatlösung eingetaucht werden. Wird anschließend das Wasser durch Trocknen entfernt, erhält man einen einsatzfähigen Absorber.

Es ist auch möglich, eine alkalische wäßrige Lösung von Aluminiumoxid mit einer wäßrigen Kaliumpermanganat-Lösung zu vermischen, das Wasser zu entfernen und den zurückbleibenden Feststoff zu trocknen und anschließend zu granulieren.

Es zeigt sich, daß es vorteilhaft ist, Granulat in Form von möglichst geringer Korngröße von 1 bis 1000 µm, bevorzugt 10 bis 800 µm, besonders bevorzugt 50 bis 500 µm zu verwenden, wogegen bei durchströmten Filtersystemen vorzugsweise grobkörnige Materialien mit einer Korngröße von 500 µm bis 5 mm eingesetzt werden. Besonders vorteilhaft ist es, feinkörniges oder gemahlenes Granulat zu verwenden. Wahlweise kann auch poröses Granulat eingesetzt werden. Die Formaldehydabsorption in geschlossenen Gebinden funktioniert auch mit grobkörnigem Material, das bei durchströmten Filtersystemen eingesetzt wird. Allerdings ist die Zeit, bis eine bestimmte Menge an Formaldehyd vernichtet ist, bei grobkörnigem Granulat um ein Vielfaches höher als bei geringer Korngröße.

Vorteilhaft wird das die Absorbermischung enthaltende Material in eine Umhüllung aus gasdurchlässigem Material verpackt, beispielsweise in ein Nylon- oder Perlonsäckchen. Es ist aber auch möglich, die Absorbermischung auf andere Weise im Inneren des versiegelten Gebindes unterzubringen, wobei lediglich auf ausreichende Diffusionsmöglichkeit der zu absorbierenden Substanzen zur Absorbermischung zu achten ist. Die Absorbermischung könnte beispielsweise auf die Innenseite des gasundurchlässigen Gebindes aufgetragen werden.

Das erfindungsgemäße Verfahren ist insbesondere vorteilhaft für die sofortige Verpackung von aus Polyacetal oder polyacetalhaltigem Material spritzgegossenen Formteilen oder von Baugruppen enthaltend solche Formteile anwendbar. Dies gilt insbesondere für Formteile oder Baugruppen, die im medizinischen Bereich (z.B. Inhalatoren, medizinische Geräte und Bestecke etc.) oder im Lebensmittel- oder sonst die Gesundheit betreffenden Bereich einzusetzen sind.

Polyacetale, d.h. Polyoxymethylenhomo- und copolymere oder -blends sind weitläufig bekannt. Ihre Herstellung, Verarbeitung und Anwendung ist hinreichend beschrieben. Auch Polyolefine, d.h. Polyolefinhomo- und copolymere oder -blends, die beispielsweise als gasundurchlässiges Verpackungsmaterial verwendet werden können, sind hinreichend bekannt und beschrieben. Dem Verpackungs-Fachmann sind zudem weitere gasundurchlässige Verpackungsmaterialien und deren z.T. mehrschichtiger Aufbau weitläufig bekannt, so daß er das erfindungsgemäße Verfahren ohne weiteres auf entsprechende geschlossene Gebinde anwenden kann.

### Beispiele

Je Versuch wurden jeweils 10 aus Polyoxymethylencopolymer (Hostaform C 9021, Ticona GmbH, Frankfurt), spritzgegossene Platten (80 x 60 x 1 mm, Gewicht einer Platte: ca. 7,1 g) sofort nach ihrer Herstellung auf einer Metallstange aufgehängt. Um zu gewährleisten, daß die Oberfläche jeder Platte mit dem Gasraum in direktem Kontakt steht, wurde jeweils zwischen zwei Platten ein Raschigring als Abstandhalter angebracht. Die gesamte Anordnung wurde mit bzw. ohne PURATEX als Absorber in einen Beutel aus Polyethylen (PE-Beutel) gebracht, der mit einem Zip-Verschluß versehen war und gasdicht verschlossen wurde.

Die Formaldehydkonzentration (FA Conc.) im Beutel wurde mit einem Dräger Quantimeter 1000 bestimmt, wobei Drägerröhrchen mit Empfindlichkeit für Konzentrationsbereiche von 0,2 bis 5 ppm und von 2 bis 40 ppm verwendet wurden.
- Versuch 1:: Keine Zugabe von PURATEX
- Versuch 2:: Zugabe von 1 g PURATEX (Granulat in gasdurchlässige Perlonfolie verpackt und als Säckchen zugegeben; mittlere Korngröße 500 µm bis 5 mm).
- Versuch 3:: Zugabe von 5 g PURATEX (Granulat in gasdurchlässige Perlonfolie verpackt und als Säckchen zugegeben; grobkörnig, mittlere Korngröße 500 µm bis 5 mm)
- Versuch 4:: Zugabe von 1 g PURATEX (Granulat in gasdurchlässige Perlonfolie verpackt und als Säckchen zugegeben; mittlere Korngröße 10 µm bis 1 mm)

**Tabelle 1:**

| Ergebnisse der Versuche 1-4 | | | | |
|---|---|---|---|---|
| Zeit [h] | Versuch 1 FA Conc. [ppm] | Versuch 2 FA Conc. [ppm] | Versuch 3 FA Conc. [ppm] | Versuch 4 FA Conc. [ppm] |
| 0 | Herstellung der Spritzgußplatten = Zeitpunkt 0 | | | |
| 1 | 10 | 20 | 10 | 15 |
| 2 | 15 | 15 | 30 | 5 |
| 3 | 15 | 5 | 5 | 3 |
| 4 | 20 | 5 | 5 | 2 |
| 5 | 25 | 5 | 5 | 2 |
| 6 | 40 | 5 | 5 | 1 |
| 22 | - | 2 | 1 | 1 |
| 30 | 35 | 2 | 1 | 1 |

Ohne Zugabe von PURATEX steigt die Formaldehydkonzentration im PE-Beutel innerhalb von 6 Stunden auf 40 ppm an und bleibt dann im Rahmen der Meßgenauigkeit konstant. Eine Zugabe von 1 g PURATEX der mittleren Korngröße 500 µm bis 5 mm führt dazu, daß die Formaldehydkonzentration nach einem anfänglichen Anstieg mittelfristig auf 5 ppm sinkt und nach 22 h einen Wert von nur noch 2 ppm erreicht. Bei Zugabe von 5 g PURATEX der mittleren Korngröße 500 µm bis 5 mm bzw. von 1 g PURATEX der mittleren Korngröße 10 µm bis 1 mm beträgt die Formaldehydkonzentration nach 22 h nur noch 1 ppm und ist somit im Bereich der Formaldehyd-Geruchsschwelle.

## Patentansprüche

1. Verfahren zur Desodorierung des Innenraums von geschlossenen, gasundurchlässigen Gebinden, wobei in das Gebinde eine geringe Menge einer Absorbermischung enthaltend aktiviertes Aluminiumoxid und Kaliumpermanganat gegeben wird.

2. Verfahren nach Anspruch 1, **dadurch gekennzeichnet, daß** die Absorbermischung wahlweise zusätzlich Aktivkohle, Wasserabsorber und/oder weitere Formaldehydfänger wie Polyalkylenimide enthält.

3. Verfahren nach Anspruch 1 oder 2, **dadurch gekennzeichnet, daß** das Gebinde formaldehydfreisetzende, insbesondere polyacetalhaltige Materialien oder Formteile enthält und sich die Konzentration von flüchtigen Substanzen, insbesondere Spaltungsprodukten wie Formaldehyd im Innenraum des Gebindes durch die Zugabe der Absorbermischung nachhaltig nicht erhöht.

4. Verfahren nach Anspruch 3, **dadurch gekennzeichnet, daß** die Konzentration von Formaldehyd im Innenraum des Gebindes nachhaltig auf maximal 5 ppm, vorzugsweise maximal 2 ppm begrenzt wird.

5. Verwendung einer Absorbermischung enthaltend aktiviertes Aluminiumoxid und Kaliumpermanganat sowie wahlweise zusätzlich Aktivkohle, Wasserabsorber und/oder weitere Formaldehydfänger wie Polyalkylenimide zur Desodorierung des Innenraums von geschlossenen, gasundurchlässigen Gebinden.

6. Verwendung nach Anspruch 5, wobei das geschlossene Gebinde ein formaldehydfreisetzendes, insbesondere polyacetalhaltiges Material oder Formteil enthält.

7. Verwendung nach Anspruch 5 oder 6, **dadurch gekennzeichnet, daß** die Konzentration von flüchtigen Substanzen, insbesondere Spaltungsprodukten wie Formaldehyd im Innenraum des Gebindes durch die Zugabe der Absorbermischung nachhaltig nicht erhöht wird.

8. Verwendung nach Anspruch 7, **dadurch gekennzeichnet, daß** insbesondere die Konzentration von Formaldehyd im Innenraum des Gebindes nachhaltig auf maximal 5 ppm, vorzugsweise maximal 2 ppm begrenzt wird.

9. Verwendung nach einem der Ansprüche 5 bis 8 zur Desodorierung von Verpackungen, die Formteile oder Baugruppen für Anwendungen im medizinischen Bereich, im Lebensmittelbereich oder sonst die Gesundheit betreffenden Bereich beinhalten.

## Claims

1. A process for deodorizing the space within closed, gastight packs, where a small amount of an absorber mixture comprising activated aluminum oxide and potassium permanganate is placed into the pack.

2. The process as claimed in claim 1, wherein the absorber mixture may, if desired, also comprise activated carbon, water absorbers and/or other formaldehyde scavengers, such as polyalkyleneimides.

3. The process as claimed in claim 1 or 2, wherein the pack comprises formaldehyde-releasing, and in particular polyacetal-containing, materials or moldings, and the concentration of volatile substances, in particular of cleavage products, such as formaldehyde, in the space within the pack is not durably increased by adding the absorber mixture.

4. The process as claimed in claim 3, wherein the concentration of formaldehyde in the space within the pack is durably restricted to not more than 5 ppm, preferably not more than 2 ppm.

5. The use of an absorber mixture comprising activated aluminum oxide and potassium permanganate, and also, if desired, activated carbon, water absorbers and/or other formaldehyde scavengers, such as polyalkyleneimides, for deodorizing the space within closed, gastight packs.

6. The use as claimed in claim 5, where the closed pack comprises a formaldehyde-releasing, and in particular polyacetal-containing, material or molding.

7. The use as claimed in claim 5 or 6, wherein the concentration of volatile substances, in particular of cleavage products, such as formaldehyde, in the space within the pack is not durably increased by adding the absorber mixture.

8. The use as claimed in claim 7, wherein in particular the concentration of formaldehyde in the space within the pack is durably restricted to not more than 5 ppm, preferably not more than 2 ppm.

9. The use as claimed in any of claims 5 to 8 for deodorizing packaging in which moldings or assemblies for applications in the medical sector, in the food and drink sector, or in another sector affecting health, are present.

## Revendications

1. Procédé de désodorisation de l'espace interne de fûts fermés, imperméables aux gaz, une quantité faible d'un mélange d'agents absorbants contenant de l'oxyde d'aluminium activé et du permanganate de potassium étant introduite dans le fût.

2. Procédé selon la revendication 1, **caractérisé en ce que** le mélange d'agents adsorbants contient, au choix, en sus, du charbon actif, des agents absorbants de l'eau et/ou d'autres agents piégeurs du formaldéhyde, comme les polyalkylènimides.

3. Procédé selon la revendication 1 ou 2, **caractérisé en ce que** le fût contient des matériaux ou des pièces préformées libérant du formaldéhyde, en particulier contenant des polyacétals et **en ce que** la concentration des substances volatiles, en particulier des produits de clivage comme le formaldéhyde n'est pas augmentée, de manière persistante, dans l'espace interne du fût à la suite de l'addition du mélange d'agents absorbants.

4. Procédé selon la revendication 3, **caractérisé en ce que** la concentration de formaldéhyde dans l'espace interne du fût est, de manière persistante, limitée au maximum à 5 ppm, de préférence, au maximum à 2 ppm.

5. Utilisation d'un mélange d'agents absorbants contenant de l'oxyde d'aluminium activé et du permanganate de potassium ainsi qu'au choix, en sus, du charbon actif, des agents absorbants de l'eau et/ou d'autres agents piégeurs du formaldéhyde, comme les polyalkylènimides, en vue de la désodorisation de l'espace interne de fûts fermés, imperméables aux gaz.

6. Utilisation selon la revendication 5, le fût fermé contenant un matériau ou une pièce préformée libérant du formaldéhyde, en particulier contenant des polyacétals.

7. Utilisation selon la revendication 5 ou 6, **caractérisée en ce que** la concentration des substances volatiles, en particulier des produits de clivage, comme le formaldéhyde, n'est pas augmentée, de manière persistante, dans l'espace interne du fût à la suite de l'addition du mélange d'agents absorbants.

8. Utilisation selon la revendication 7, **caractérisée en ce que** la concentration de formaldéhyde dans l'espace interne du fût est, de manière persistante, limitée au maximum à 5 ppm, de préférence, au maximum à 2 ppm.

9. Utilisation selon l'une quelconque des revendications 5 à 8, en vue de la désodorisation d'emballages, qui contiennent des pièces préformées ou des modules de construction pour des applications dans le domaine médical, dans le domaine des produits alimentaires ou sinon dans le domaine concernant la santé.
